Europäisches Patentamt

European Patent Office

Office européen des brevets

(11) Publication number: **0 083 848**
**A1**

## (12) EUROPEAN PATENT APPLICATION

(21) Application number: **82306507.3**

(22) Date of filing: **07.12.82**

(51) Int. Cl.³: **A 01 K 29/00**
**A 61 B 5/10**

(30) Priority: **11.12.81 GB 8137419**

(43) Date of publication of application:
**20.07.83 Bulletin 83/29**

(84) Designated Contracting States:
**AT BE CH DE FR GB IT LI LU NL SE**

(71) Applicant: **HRH INDUSTRIES & TRADING LIMITED**
**Stadle 36**
**FL-9490 Vaduz(LI)**

(72) Inventor: **Metcalf, Peter Edward Anthony**
**Jubilee House Topcliffe**
**Nr. Thirsk North Yorkshire(GB)**

(74) Representative: **Wharton, Peter Robert et al,**
**URQUHART-DYKES & LORD Beckett's Bank Chambers**
**19 Cheapside**
**Bradford BD1 4HR(GB)**

(54) **Method and apparatus for assessing the capabilities of a horse.**

(57) The method comprises calculating the full adult height of the horse, filming the horse in motion, calculating the proportions of adult lengths of parts of the horse's legs and body, processing said film and calculated information such that it is in a form which can be compared with corresponding information and data of an ideal model horse, the model being derived from physiological assessment of horses of known capabilities, the comparison being such as to enable an assessment of the subject horse's physiological capabilities to be made. The apparatus may comprise a chamber (3) having a track about which the horse may be moved, the chamber being capable of being tilted at an angle to the horizontal so as to vary the load conditions under which the horse operated. Force platforms are provided to record the force exerted by the horse's hooves, and cameras (30 etc.) are provided to record the motion of the horse under varying conditions.

FIG.1.

- 1 -

## Physiology

The present invention relates to the physiological capability of a horse, especially a racing horse.

Within the animal breeding and training world, more especially the horse-racing world, it is customary to assess the potential of young animals by taking into consideration the pedigree of the animal, its temperament, and its visual appearance. However, especially in the horse-racing world, where foals and young horses can be extremely expensive if they have a pedigree in which known good race horses appear, this method is not wholly satisfactory, as a distinguished pedigree is no guarantee that the horse will be as good as its predecessors.

According to the present invention there is provided a method of assessing the physiological capability of a horse, the method comprising calculating the full adult height of the horse, filming the horse in motion, calculating the proportions and adult lengths of parts of the horses legs and body, and processing said film and calculated information such that it is in a form which can be compared with corresponding information and data on an ideal model horse, the model being derived from physiological assessment of horses of known capabilities, the comparison being such as to enable an assessment of the subject horses' physiological capabilities to be made.

The present invention thus provides a method for assessing the potential capabilities of a horse, and can be potentially advantageous for use with young horses. In such a case the full adult height may be estimated by a procedure involving measurement of the present height of the horse, the calculation of the horse's exact age in days, and from x-ray pictures of the horse's knees, the growth regions of the leg can be identified and an estimate of the full adult height of the horse be made.

Preferably the comparison of the data derived from the subject horse is compared with the corresponding data for the model horse by suitable computing means, which may be a microprocessor, a microcomputer, a mini computer or a main frame computer depending on the capacity required.

In order to obtain the most accurate assessment of the horse's capabilities, it is desirable that the horse be filmed from a variety of angles at a variety of gaits. Preferably the horse is filmed from the front, the side, and the rear, at a walk, trot, canter and a gallop.

The above information allows an assessment of the mechanical efficiency of the movements of the horse to be made, but for a full physiological assessment it is desirable that other information is collated.

Preferably there is taken from the horse a sample of muscle fibre from the hip. The sample is then analysed in order to ascertain the relative proportions of white and red muscle fibres. In order for the horse to become a good race horse, it is desirable that there is a relatively high proportion of white muscle fibres which give the horse speed, whilst the red fibres give stamina.

It is advantageous that the respiration rate and heart beat rate of the horse under a variety of conditions are also monitored. Desirably the proportion of oxygen breathed by the horse is calculated by comparing a sample of ambient air with a sample of the air breathed out of the horse. This may relatively easily be done by placing a close fitting mask over the horse's nose and mouth, with an outlet pipe from the mask to the analysing means. The air breathed in by the horse can then be monitored and compared with the air breathed out by the horse so as to give the amount or the proportion of oxygen taken up from the air by the horse. Desirably this should be as high a proportion as possible. Conveniently the same close fitting mask includes transistorised cells which may measure the respiration rate and the heart beat rate of the horse.

Preferably the full body weight of the horse should be measured, and more preferably the distribution of weight should be measured. It is also desirable that an estimate of the relaxation and extension capacities of the muscles be made. This information allows a more accurate assessment of the mechanical efficiency of the horse to be made, but in order to assess such efficiency more fully it is desirable that information be collated regarding the force applied to the ground by each hoof at various gaits, and the time each hoof is in contact with the ground at the various gaits, be monitored as these give an indication of the power of the muscles of the horse.

Using information such as that described above,

it is possible to calculate the probable maximum speed of the horse, and also how far the horse would be able to run in a given time, i.e. its top average speed. Information of this sort would then allow the potential of young horses to be better assessed, and would help in the breeding of horses having desirable characteristics.

The present invention also provides within its scope apparatus for assessing the physiological capability of a horse, comprising filming means for recording the movements of the horse, means for calculating the full adult height of the horse and the relative proportions and adult lengths of parts of the horse's body, processing means for preventing the information in a form suitable to form a comparison with corresponding information of an idealised model horse, comparison means for carrying out the comparison, and presentation means for presenting the results of the comparison so that assessment of the physiological capability of a subject horse may be made.

According to the present invention there is further provided an apparatus for assessing the physiological capability of a horse which comprises a chamber having on its floor a track portion about which a horse may be moved, the track being equipped with a telemetric force platform or other load transducer capable of calculating the force applied to it by the horse's hooves and the chamber being pivotally mounted so that the floor of the chamber and therefore the track portion can be tilted at an angle to the horizontal.

Preferably the chamber can be tilted up to an angle of 35° from the horizontal. Preferably the chamber is fitted with a number of cameras to record the motion of the horse about the track, which may be substantially rectangular, be covered in a resilient rubber coating, and may have a grid pattern to aid measurements. The cameras may be mounted independently of the chamber so as to prevent vibration induced by the movement of the horse interfering with the quality of the pictures obtained.

An embodiment of the present invention will now be described by way of example only and with reference to the accompanying drawings in which:-

Figure 1 is a vertical cross-section through a building incorporating apparatus in accordance with the present invention;

Figure 2 is a top plan view of the building of Figure 1; and

Figure 3 is a top plan view of another embodiment of the apparatus in accordance with the present invention.

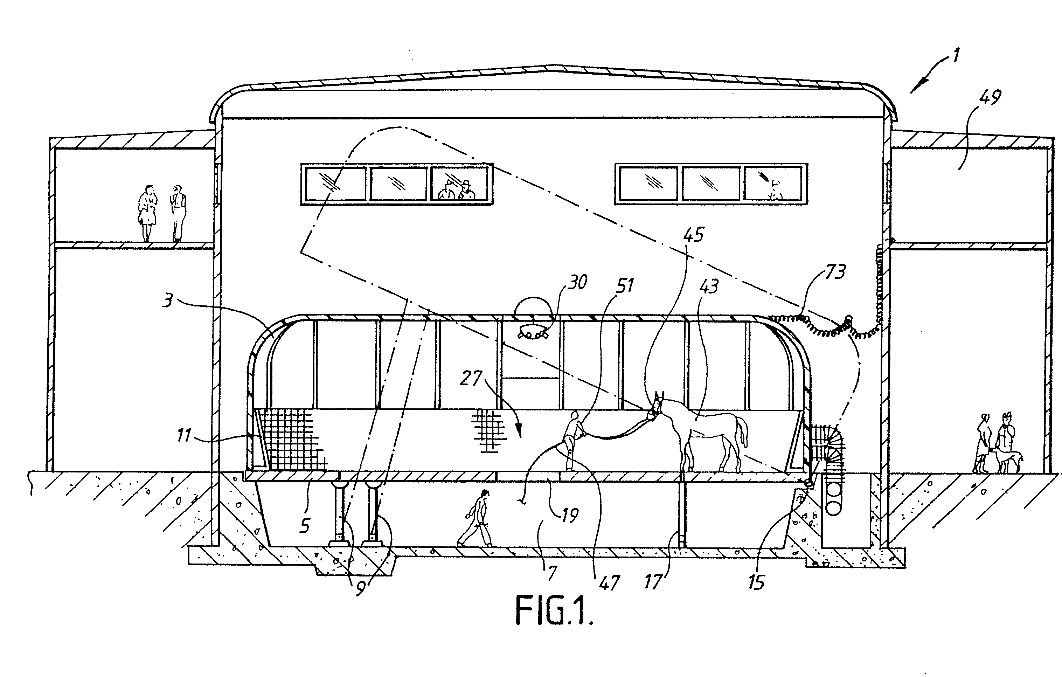

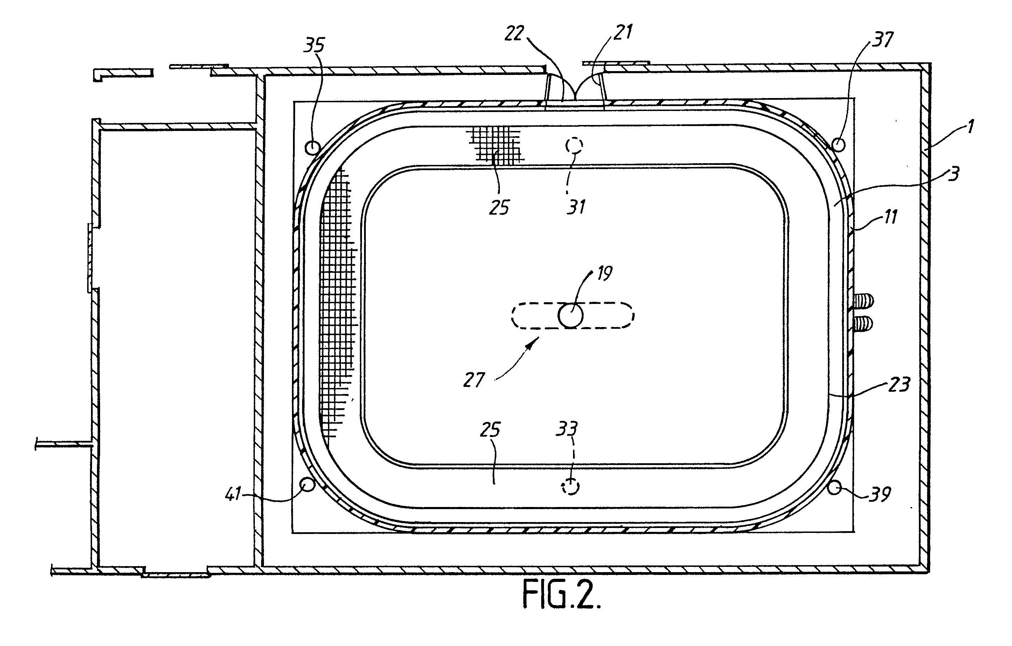

Referring particularly to Figures 1 and 2 of the accompanying drawings, apparatus in accordance with the present invention is contained within a building 1. Within the building 1 is an enclosed chamber 3 equipped with an air-conditioning supply. The base of the chamber 3 is substantially at ground level and is formed of a plurality of floor sections 5. The floor sections 5 are suspended over a maintenance pit 7. Extending between the floor of the maintenance pit 7 and floor sections 5 are four telescopic jacks 9 which may be extended so as to pivot the chamber 5 about a pivot point 15 at one end thereof so that the floor of the chamber 3 can lie at any angle up to approximately 35$^{o}$ from the horizontal. When in the lowered position the chamber is supported by means of fixed supports 17 as well as by telescopic jacks 9.

The chamber 3 is provided with inner sloping walls 11 which are covered with resilient rubber material so as to protect the horse from serious injury should the horse collide with the walls. The walls 11 are provided with ventilation grills leading to the air-conditioning extractor and supply. The walls are also provided with a grid pattern. The upper part of the chamber is covered by a perspex wall and roof. There is provided in the central region 27 of the floor of the chamber 3 a hole 19 connecting the chamber 3 to the pit 7.

Referring now more particularly to Figure 2 of the accompanying drawings, a door 21 in the exterior of the building 1 and a corresponding door 22 in the side walls of the chamber 3 allow access from the chamber 3 to the exterior of the building 1. The peripheral region 23 of the floor 5 of the chamber 3 is in the form of a substantially rectangular track about which a horse may be moved, the track being covered with resilient rubber coating painted green so as to resemble grass but with a grid system corresponding to that provided on the lower side walls 11 of the chamber 3. The longer straight sections 25 of the substantially rectangular track 23 are equipped with tele-metric force platforms. These platforms are capable of calculating the force applied to them in a digital form suitable for subsequent processing.

The chamber 3 is fitted with a number of cameras. In substantially the centre of the top of the chamber 3 is a rotatable mounting/carrying 30 an electronic video camera and an infra-red camera. These cameras produce signals in digital form which can be recorded, processed, and replayed. Suspended over the straight sections 25 of the track 23 are video cameras 31 and 33. At each corner of the chamber 3 positioned on the outside curves of the corners of the track 23 are cameras 35, 37, 39 and 41.

The output from all the cameras and from the tele-metric force platforms are carried by means of electrical connections 73 to computing means enclosed in an adjacent office 49.

In use, a horse 43 led by a trainer 51 is brought into the chamber 3. The horse 43 is fitted with a close fitting mask 45 over the nose and mouth.

The mask 45 is provided with transistorised transducers which can detect the respiration rate and heart beat rate of the horse. The transducers have electrical connections, which, together with an air supply pipe 47 lead from the mask 45 to the central region 27 of the floor of the chamber 3, where they exit from the chamber 3 through the hole 19 in the floor of the chamber. They then subsequently pass through the pit 7 and are lead up to the computing means in the office 49.

On entering the chamber 3 the horse is stood on the tele-metric force platform 25 nearest the door 22 so that its weight can be recorded. It is then walked round the track 23 on a lunge rope with the trainer 51 in the central region 27 of the chamber 3. Whilst the weight of the horse is being recorded, the cameras 35 and 37 looking along the length of the tele-metric force platform 25 take views of the front and the rear of the horse, the roof-mounted cameras 31 and 30 film the side of the horse and top of the horse. These pictures can then be processed so that the exact dimensions of the horse can be calculated from the grid markings on both the walls of the chamber 3 and the track 23/of the chamber 3.

The horse is moved around the track 23 at a variety of gaits. The horse is first walked, then trotted, then cantered and then galloped around the track, with the cameras filming all its movements and the tele-metric force platforms calculating each impact of the hoof on the relevant section of the track. In combination the film from the cameras, which can give the time that each hoof is in contact with the ground, and the tele-metric force platform 25 which gives the force of each impact of the hoof on the ground, can yield information as to the power of the muscles of the horse.

When the horse has been analysed moving on the flat, the floor of the chamber 3 is pivoted about the point 15 by the raising of the telescopic jack 9. These may raise the chamber to any desired angle up to $35^{\circ}$. The recording of the horse's movements are repeated with the chamber in the inclined positions, so putting more strain on the horse. The information is transmitted to the computing equipment in the office 49.

Whilst information about the mechanical movements of the horse are being gained by the cameras and the tele-metric force platforms, the respiration rate and the heart beat rate of the horse are also being recorded and analysed. In addition, the air breathed by the horse through the pipe 47 to the mask 45 is analysed to calculate the proportion of oxygen taken up by the horse from the air that it breathes. The anaylsis equipment is in the office 49 and feeds its information to the computing means also in the office 49.

Apparatus in accordance with the present invention thus provides means by which the physiological capabilities of a horse may be assessed by comparison with those of an idealised model horse, so improving the chances of producing a good race horse.

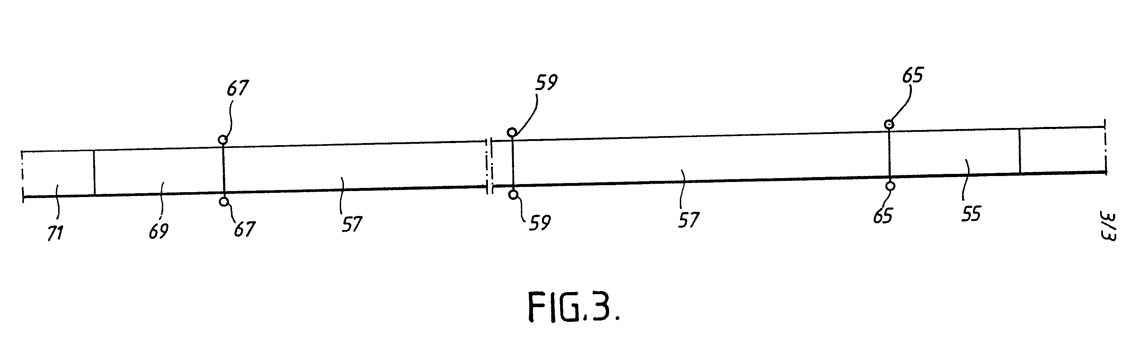

Figure 3 is a top plan view of further apparatus in accordance with the present invention. The apparatus comprises a galloping track approximately 80 yards long. The track is positioned on the end of, for instance, a one mile length of ordinary galloping track. In this embodiment, the normal track is composed of sand and wood shavings and is, together with the further 80 yards of the track fully enclosed so as to be weather-proof.

The prepared length of the track commences at the end of the normal galloping track by the joining to the normal track of a 10 yard length of track having a rubberised surface. This section is denoted by number 55 of Figure 3. After the 10 yard section of 55, there is a 60 yard section 57 of the same or similar surface material, but provided with tele-metric pressure cells over the whole length thereof. At the end of the 60 yard pressure pad section 57 is a further 10 yard section 69 without pressure pads, before the gallop reverts to the normal surface 71.

At the interface between the 10 yard opening of the rubber      section 55 and the pressure pad section 57 are photo-electric cells 65.  The cells generate and detect a beam of light passing across the gallop.  At the opposite end of the gallop section 57 at its junction with section 69 is a corresponding set of photo-cells 67.  The photo-cells are connected to timing and recording equipment so that at the start of the pressure pad section 57, when in use, a horse breaks the light beam between the photo-cells 65 this starts  timing equipment.  On reaching the end of the pressure section 57 the horse again breaks a beam between the cells 67 stopping the timer and recording equipment.  This enables the average speed of the horse over the 60 yard section of the track 57 to be calculated.

At the mid-point of the pressure pad section 57 are a pair of rotatable cameras 59 with high shutter speeds of approximately 5,000 frames a second.  These are positioned on each side of the gallop and can be controlled so as to pan along the length of the gallop to keep the horse in view throughout.  The information recorded by the cameras, in combination with the data received from the pressure cells underneath the section 57 of the gallop enables data to be gained as to the force applied by each hoof to the ground, the time that each hoof is in contact with the ground, and the maximum speed reached by the horse during the 60 yards.  This renders valuable information about the power generated by the horse's muscles.

For overall monitoring of the horse during its progress over the gallop, an electronic video camera is suspended above the mid-point of the pressure section of the gallop 57.

In use, the horse would be ridden along a, for instance, one mile section of the gallop of a normal surface, and then upon reaching the beginning of the prepared section of the gallop is encouraged to full speed. The first section of the gallop without pressure pads is to allow the horse to settle down after the change in the surface of the gallop, before taking recordings of the force applied by the horse to the ground. The horse sprints over the 60 yard distance of the pressure track 57, has its movements recorded and analysed, and can then relax to a normal pace on the end of the prepared track where it re-joins normal surface.

The present invention thus provides method and apparatus for testing, assessing, and monitoring the physiological capabilities of the horse from a young age, and during its training as a race horse.

The track 23 may be provided with a movable mechanical barrier which may be driven round the track at any desired speed. This may be used to 'work' the horse and allows the horse to be worked at a constant speed which can be very accurately determined. The movable barrier can be set to various working paces such as walking, trotting, cantering, galloping, etc. Furthermore, the barrier may be provided with a stimulation unit in order to keep the horse moving, for example an audible and/or visual alarm such as a buzzer and/or flashing light.

CLAIMS:-

1. Apparatus for assessing the physiological capability of a horse which comprises filming means for recording the movements of the horse, means for calculating the full adult height of the horse and the relative proportions and adult lengths of parts of the horses body, processing means for presenting the information in a form suitable to form a comparison with corresponding information of an idealised model horse, comparison means for carrying out a comparison, and presentation means for presenting the results of the comparison so that assessment of the physiological capability of the subject horse may be made.

2. An apparatus as claimed in claim 1 in which the filming means comprise photographic and/or video cameras.

3. Apparatus as claimed in either of claims 1 or claim 2 in which the means for calculating, the means for processing and the means for comparison and the means for presentation comprise a micro-processor, micro-computer, mini-computer or main frame computer.

4. A method of assessing the physiological capability of a horse which comprises calculating the full adult height of the horse, filming the horse in motion, calculating the proportions and adult lengths of parts of the horses legs and body, processing said film and calculated information such that it is in a form which can be compared with corresponding information and data on an ideal model horse, the model being derived from a physiological assessment of horses of known capabilities, the comparison being such as to enable an assessment of a subject horses physiological capabilities to be made.

5. A method as claimed in claim 4 in which the full adult height is estimated by measuring the present height of the horse, identifying the growth region of the legs by means of x-ray pictures, ascertaining the horse's exact age, and making an estimate based on this data.

6. A method as claimed in either of claims 4 or 5 in which a sample of muscle fibre is taken from the hip of the horse and analysed in order to ascertain the relative proportions of widespread muscle fibres.

7. A method as claimed in any of claims 4 to 6 in which the respiration rate and heartbeat of the horse is monitored under a variety of conditions.

8. An apparatus for assessing the physiological capability of a horse which comprises a chamber having on its floor a track portion about which a horse may be moved, the track being equipped with a telemetric force platform capable of calculating the force applied to it by the horse's hooves, and the chamber being pivotally mounted so that the floor of the chamber and therefore the track portion can be tilted at an angle to the horizontal.

9. An apparatus as claimed in claim 8 in which the chamber can be tilted at an angle of up to $35^{O}$ from the horizontal.

10. An apparatus as claimed in either of claims 8 or 9 in which the track is substantially rectangular, covered in a resilient rubber coating, and having a grid pattern to aid measurements.

11. An apparatus as claimed in any one of claims 8 to 10 in which the chamber is fitted with a number of cameras to record the motion of a horse about the track.

FIG.1.

FIG.2.

FIG.3.

0083848

## DOCUMENTS CONSIDERED TO BE RELEVANT

| Category | Citation of document with indication, where appropriate, of relevant passages | Relevant to claim | CLASSIFICATION OF THE APPLICATION (Int. Cl. ³) |
|---|---|---|---|
| A | US-A-4 233 845  (PRATT) | | A 01 K   29/00<br>A 61 B    5/10 |
| A | FR-A-1 564 781  (LEPAUTE) | | |
| A | JOURNAL OF ENGINEERING FOR INDUSTRY, vol. 97, no. 1, February 1975, pages 49-57; J.A.BARTZ  et al.: "Computer program  to generate dimensional and inertial  properties of the human body". | | |
| A | US-A-3 999 611  (BUCALO) | | |
| A | US-A-3 894 437  (HAGY) | | TECHNICAL FIELDS SEARCHED (Int. Cl. ³)<br><br>A 01 K<br>A 61 B |
| A | FR-A-2 462 179  (BERGERET) | | |

The present search report has been drawn up for all claims

| Place of search | Date of completion of the search | Examiner |
|---|---|---|
| THE HAGUE | 18-03-1983 | VILBIG K |